# EUROPEAN PATENT SPECIFICATION

(11) **EP 2 131 770 B1**
(45) Date of publication and mention of the grant of the patent: **22.06.2016**
(21) Application number: 08735156.5
(22) Date of filing: 10.04.2008
(51) Int. Cl.: A61B 17/88, B25B 23/10, A61B 17/86

(54) **BONE SCREW HOLDING DEVICE**
KNOCHENSCHRAUBENHALTEVORRICHTUNG
DISPOSITIF DE FIXATION POUR VIS À OS

(30) Priority: 10.04.2007 US 922599 P
(43) Date of publication of application: 16.12.2009
(73) Proprietor: Stryker European Holdings I, LLC, Kalamazoo, MI 49002 (US)
(72) Inventor: SCHWAGER, Manuel, CH-8057 Zürich (CH); DORAWA, Klaus, 24232 Schönkirchen (DE)
(74) Representative: Schmitz, Alexander
(86) International application number: PCT/EP2008/002847
(87) International publication number: WO 2008/122446

(56) References cited:
- WO-A-2007/006282
- WO-A-2007/021850
- DE-U1-202005 012 781
- US-A- 4 911 593
- US-A- 5 139 499
- US-A1- 2003 074 003
- US-A1- 2007 010 816
- US-B1- 6 286 401

## Description

### Background of the invention

### Field of Invention

The present invention relates to a device for inserting bone screws during a surgical procedure. More particularly, the present invention relates to an improved system of a bone screw holding device and a bone screw wherein the bone screw is held rigidly prior to and during insertion.

### Description of the related art

Many types of bone screws are available for use with specific type of bone tissue or orthopedic implants. Often, a surgeon needs to install a large number of bone screws. Bone screws that are not secured to the driver can slip and become lost in the tissue. This results in unnecessary and undesirable increase in operating time. Therefore, it is desirable for the bone screws to be coupled to the driver.
Prior art screw and driver system that capture the screw prior to insertion do not provide rigid attachment of the screw to the driver and therefore, have the same shortcoming as the traditional screw and screw driver. Accordingly, there is a need for a bone screw holding device that rigidly engages the bone screw prior to and during the insertion.

US 5,139,499 A describes a screw and driver combination designed so that the screw is releasably axially couplable with the driver and so that the screw is positively rotatably engageable with the driver. The screw comprises an axial bore of circular cross-section and a counter bore of polygonal cross-section. The driver comprises a radially-compressible front portion sized to engage the circular axial bore of the screw with a minor spring interference fit and an intermediate portion sized to positively rotatably engage the polygonal counter bore of the screw.

US 6,286,401 B1, as well as US 2007/0010816 A1 and WO 2007/021850 A1, discloses a screw driver comprising a driver bit with a drive portion for engaging a socket within a socket-headed screw, with the drive portion being expanded by the distal end of an internal pushrod operating on the conical surface of a hold within the drive bit.

WO 2007/006282 A2 discloses a screw driver for an inner profile, wherein the driver head includes two driver parts with a slot between these parts, so that, when the driver parts are pressed from the outside by a bushing, the driver parts bend towards each other. The bended driver parts can be introduced into the inner profile of a screw. Releasing the driver parts results in a press fit of the driver parts with the surface of the inner profile.

DE 20 2005 012 781 U1 describes an instrument for holding a bone screw at the outer surfaces of the screw head. The gripping arms of the instrument are adapted to be pressed towards each other to securely hold the bone screw.

US 2003/0074003 A1 and US 4,911,593 disclose screw heads with additional deflectable means for engagement with a tool portion of a screw driver.

### Summary of the invention

The present invention overcomes the shortcomings of the prior art by providing a screw holding device. As used herein, when referring to the screw holding device or its components, the term "proximal" means closer to the user of the screw holding device (i.e., the surgeon) and the term "distal" means more distant from the user of the screw holding device (i.e., the surgeon).

The invention is disclosed in independent claim 1 with preferred embodiments disclosed in the dependent claims.

In general, an orthopedic screw holding device for holding a bone screw according to the invention, comprises: a screw engaging part including a distal end and a proximal end, the screw engaging part having a first portion and a second portion; wherein the screw engaging part is configured to engage with a head of the bone screw; at least one engaging means formed at the distal end, the engaging means being configured to engage a corresponding engaging means of the head of the bone screw; and a fixation slide slidably engaged with the screw engaging part and movable between a first position and a second position, wherein the first portion and the second portion are movable into a first position with the first portion and the second portion engaged with the head of the bone screw when the fixation slide is located in its first position, and the first portion and the second portion are movable into a second position when the fixation slide is located in its second position with the engaging means engaged in the corresponding engaging means of the head of the bone screw so that the orthopedic screw holding device is locked with the head of the bone screw.

Thus, an improved fixation may be provided to apply a screw. The screw holding device allows to securely fix the screw onto the screw holding device and to apply forces for mounting the screw. Thus, ist is possible to fulfill a plurality of tasks, namely to hold the screw, to fix before mounting and to release the screw after mounting, to apply rotational forces during mounting without any impact on the holding function, owing to the different direction of the rotational screw-forces and the transversal direction for holding and the radial direction for fixing.

According to an exemplary embodiment of the invention an orthopedic screw holding device further comprises at least one first facet formed at the distal end, wherein the first facet is configured to engage with a head of the bone screw.

The facets allow to apply rotational forces from the holding device to the screw. The facets may be plane, convex or concave, depending on the required forces and the available space.

According to an exemplary embodiment of the invention the fixation slide is located in its first position towards the proximal end of the screw engaging part and is located in its second position close to the distal end of the screw engaging part.

Thus, a double securing effect may be achieved when applying forces to the screw at a mounting procedure. The force for mounting the screw will be towards the bone and therefore will correspond with the moving direction of the sleeve for securing the screw with the screw holding device.

According to the invention the screw engaging part having a slot formed at the distal end, the slot extending towards the proximal end thereby forming the first portion in form of a first arm and the second portion in form of a second arm.

The slot solution provides for a space saving arrangement without pivotably mounted parts. Instead of, the first and second portion or arm may be bended, avoiding any mechanically problematic joints between the arms and the basic body of the engaging part.

According to an exemplary embodiment of the invention the first facet is configured to engage in the head of the bone screw.

Engaging in the head provides for a solution without radially protruding parts of the coupled device. Radially protruding parts may lead to problems during the surgical procedure, i.e. when crossing narrow tissue.

According to the invention the fixation slide comprises a sleeve with ribs formed on an inner surface of the sleeve for engagement with the slot formed at the distal end of the screw engaging part.

The sleeve provides for an integral construction allowing a guiding arrangement between the engagement part and the fixation slide of fixation part. Further, radially protruding portions may be avoided leading to a saver surgical procedure.

According to an exemplary embodiment of the invention the engaging means comprises a protrusion or a hook.

The protrusion or hook allows to secure the engaging part with the screw in order to avoid loose of the screw during application. Thus, forces in both directions, push and pull, ar possible without the risk of decoupling the holder and the screw.

According to an exemplary embodiment of the invention the first arm and the second arm in the first position can be moved towards each other.

This allows to insert the engaging part of the screw holder in the counterpart of the screw head even if a protrusion and a notch are provided in the recess of a screw head and a tip portion of the holding device.

According to an exemplary embodiment of the invention an orthopedic screw holding and insertion system comprises a bone screw having a head, the head having an engaging means and an inventive bone screw holding device as described above.

Thus, not only a screw holding device, but also a complete system of a screw holding device and a corresponding screw may be provided.

According to an exemplary embodiment of the invention the bone screw further comprises a shank connected to the head, and threads formed on at least a portion of the shank.

Thus, the fixation of the screw may be improved.

According to an exemplary embodiment of the invention the engaging means comprises a recess formed in the head, at least a portion of the recess having at least one facet formed therein.

Thus, rotational forces may be transmitted from the Screw holder device to the screw, which allows to mount the screw with the holding device. The holding device in this case does not only serve as a holding device, but also as a tool for application.

According to an exemplary embodiment of the invention, the recess having a notch formed in the recess.

Thus, an improved and secured coupling and fixing of the screw holding device and the screw may be provided in order to transmit forces by pulling as well as by pushing.

For a better understanding of the invention, a method of applying an orthopedic screw holding device to a bone screw is described, which comprises moving a fixation slide of a orthopedic screw holding device into a first position to move a first portion and a second portion of a screw engaging part of the orthopedic screw holding device in a first position in which the first portion and the second portion can be engaged with a head of a bone screw; engaging the first portion and the second portion with a head of the bone screw; moving the fixation slide into a second position to move the first portion and the second portion of the screw engaging part in a second position in which an engaging means of the orthopedic screw holding device can be engaged in a corresponding engaging means of the head of the bone screw so that the orthopedic screw holding device is locked with the head of the bone screw; moving the fixation slide of the orthopedic screw holding device into the first position to move the first portion and the second portion of the screw engaging part of the orthopedic screw holding device in the first position in which the first portion and the second portion can be released from a head of a bone screw; releasing the first portion and the second portion from a head of the bone screw.

The screw holding device may have a screw engaging part and a fixation sleeve. The screw for use with the screw holding device may have a head and a shaft with threads. The head of the bone screw may have a recess with six facets. Towards the distal end (i.e., towards the leading edge of the screw) of the recess and continuous with the six facets may be a notch.

The screw engaging part may be tubular and may have at least one slot formed towards its distal end. The slot may separate the tubular part into at least two halves that can flex towards and away from each other. The distal ends of the at least two halves may have six facets to match the six facets in the recess. A hook may be formed on each half of the screw engaging part distal to the six facets. The hook may be shaped to fit in the notch when screw engaging part is inserted in the head of the screw. A handle may be formed on the proximal end of the screw engaging part.

The fixation sleeve may have a distal end, a proximal end and a tubular body connecting the distal end and the proximal end. The fixation sleeve may be hollow, the hollow portion may have a diameter sufficiently large to allow the screw engaging part to smoothly slide in the fixation sleeve. A disk may be formed at the proximal end of the fixation sleeve, which may be used to assist in pushing or pulling the fixation sleeve with respect to the screw engaging part.

The screw engaging part and the fixation sleeve may be assembled together to form the screw holding device. In the assembled state, the screw engaging part and the fixation sleeve may slide relative to each other. When the fixation sleeve is moved towards the proximal direction, the two halves of the screw engaging part may be able to flex easily towards each other. When the fixation sleeve is moved towards the distal end of the screw engaging part, the two halves of the screw engaging part may be prevented from flexing towards each other.

In use, the fixation sleeve may slide in the proximal direction and the distal tip of the screw engaging part may be pressed against the edges of the recess, thereby inserting the distal tip of the screw engaging part in the recess. When the distal tip of the screw engaging part is pressed against the edges of the recess the two halves of the screw engaging part may be pushed towards each other. With the two halves pushed towards each other allowing the distal tip of the screw engaging part the ability to enter the recess. As the tip of the distal part of the screw engaging part may travels further in the distal direction into the recess, it may reach the notch and the hooks may engage in the notch allowing the two halves of the screw engaging part to flex away from each other. The seating of the hooks in the notch may prevent the screw holding device from disengaging from the screw accidentally. At this point the fixation sleeve may be positioned distally as far as possible to prevent the two halves of the screw engaging part from flexing towards each other and thereby ensuring that the screw is securely attached to the screw holding device.

According to a further exemplary embodiment of the invention an orthopedic screw holding and insertion system comprises a bone screw having a head, a shank connected to the head, and threads formed on at least a portion of the shank, the head of the bone screw having a recess formed in the head and a notch formed in the recess, at least a portion of the recess having at least two facets formed therein; a bone screw holding device having a screw engaging part including a distal end and a proximal end, the screw engaging part having a slot formed at the distal end, the slot extending towards the proximal end thereby forming a first and a second arm on the screw engaging part ; at least two first facets formed at the distal end, the first facets configured to engage the facets in the recess; at least one hook formed at the distal end, the hooks being configured to engage the notch formed in the recess; and a fixation sleeve slidably engaged with the screw engaging part, wherein the first arm and the second arm can be moved towards each other when the fixation sleeve is located towards the proximal end of the screw engaging part to allow insertion of the first arm and the second arm in the recess and the first arm and the second arm are locked in the recess when the fixation sleeve is located close to the distal end of the screw engaging part with the first and the second arm inserted in the recess.

According to a further embodiment, an orthopedic screw holding device for holding a bone screw comprises a screw engaging part including a distal end and a proximal end, the screw engaging part having a slot formed at the distal end, the slot extending towards the proximal end thereby forming a first and a second arm; at lease two first facets formed at the distal end, the first facets configured to engage in a head of the bone screw; at least one hook formed at the distal end, the hook being configured to engage in the head of the bone screw; and a fixation sleeve slidably engaged with the screw engaging part, wherein the first arm and the second arm can be moved towards each other when the fixation sleeve is located towards the proximal end of the screw engaging part to allow insertion in the head of bone screw and the first beam and the second beam are locked in the head of the bone screw when the fixation sleeve is located close to the distal end of the screw engaging part with the first and the second arm inserted in the head of the bone screw.

A method of inserting a bone screw may comprise providing a bone screw, the bone screw having a shank connected to the head, and threads formed on at least a portion of the shank, the head of the bone screw having a recess formed in the head and a notch formed in the recess, at least a portion of the recess having at least two facets formed therein; providing a bone screw holding device, the bone screw holding device having: a screw engaging part including a distal end and a proximal end, the screw engaging part having a slot formed at the distal end, the slot extending towards the proximal end thereby forming a first and a second arm on the screw engaging part; at lease two first facets formed at the distal end, the first facets configured to engage the facets in the recess; at least one hook formed at the distal end, the hooks being configured to engage the notch formed in the recess; and a fixation sleeve slidably engaged with the screw engaging part; sliding the fixation sleeve towards the proximal end of the screw engaging part; inserting the first and the second arms in the recess; sliding the fixation sleeve towards the distal end of the screw engaging part; turning the screw holding device to insert the bone screw in a bone; sliding the fixation sleeve towards the proximal end of the screw engaging part; and releasing the screw holding device from the screw.

It should be noted that the following described exemplary embodiments of the invention apply for the device, the system and a method, which method is described for a better understanding of the invention.

It should be noted that the above features may also be combined. The combination of the above features may also lead to synergetic effects, even if not explicitly described in detail.

These and other aspects of the present invention will become apparent from and elucidated with reference to the embodiments described hereinafter.

### Brief description of the drawings

Exemplary embodiments of the present invention will be described in the following with reference to the following drawings.
Figure 1 is a cross sectional view of a screw holding device engaged with a screw.
Figure 2 is an enlarged view of a portion for figure 1 showing details of engagement between the screw holding device and the screw.
Figure 3 is a cross sectional view of the screw of figure 1.
Figure 4 is an enlarged view of the head portion of the screw of figure 3.
Figure 5 is an isometric view showing a screw engagement part.
Figure 6 is an enlarged view of the distal tip of the screw engagement part of figure 5.
Figure 7 is a side view of the screw engagement part of figure 5.
Figure 8 is an enlarged view of the distal tip of the screw engagement part of figure 7.
Figure 9 is an isometric view of a fixation sleeve.
Figure 10 is an enlarged view of the distal tip of the fixation sleeve of figure 9.
Figure 11 is an isometric view of a screw holding device engaged with a screw.
Figure 12 is a side view of a screw.
Figure 13 is an isometric view of a screw.
Figure 14 is an isometric view of a screw engagement part.
Figure 15 is an isometric view of a screw holding device.
Figure 16 is an isometric view of a fixation sleeve.
Figure 17 is another isometric view of a fixation sleeve.
Figure 18 is a view of an assembled model of the screw holding device and a screw.
Figure 19 is a view of a de-assembled model of the screw holding device and a screw.
Figure 20 is a cross-sectional view of an assembled model of the screw holding device and a screw.
Figure 21 is a cross-sectional view of a coupled model of the screw holding device with a screw.
Figure 22 is a perspective view of an assembled model of the screw holding device and a screw.
Figure 23 is a view of a further embodiment of a coupled model of the screw holding device with a screw.
Figure 24 is a cross-sectional view of Figure 23.
Figure 25 is a perspective view of a detail of a coupled model of the screw holding device with an opaque screw.

### Detailed description

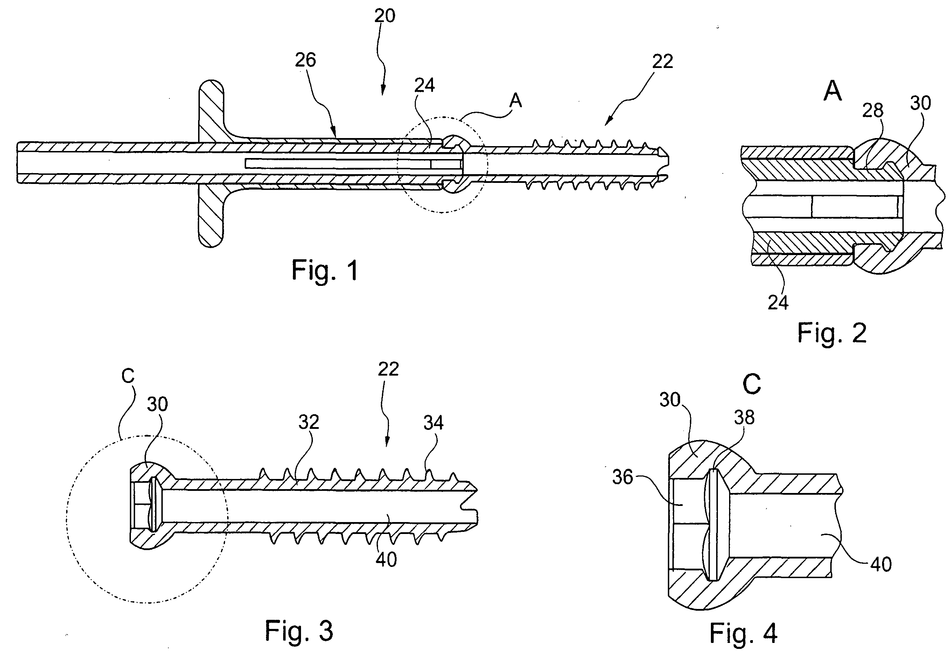

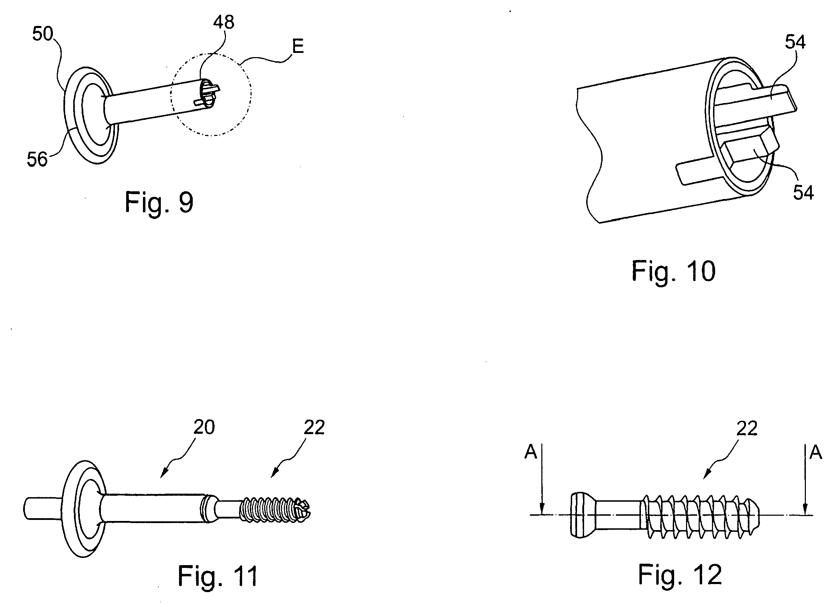

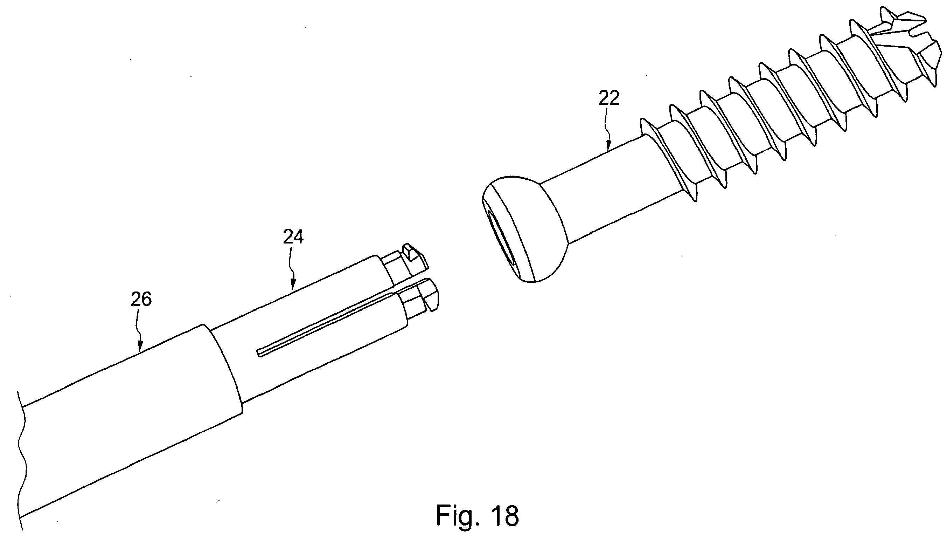

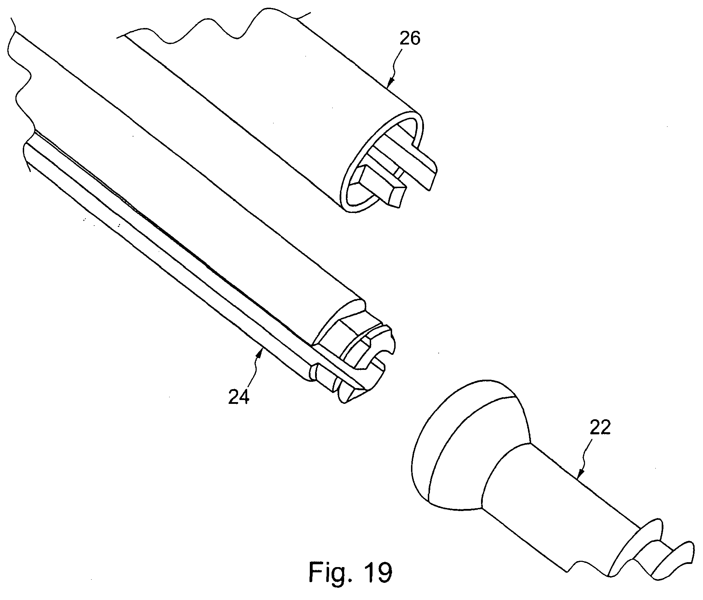

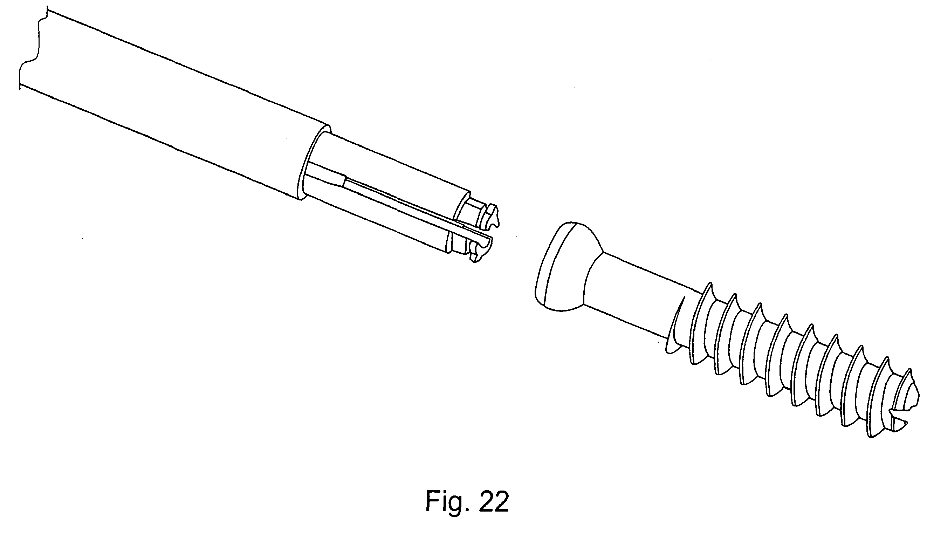

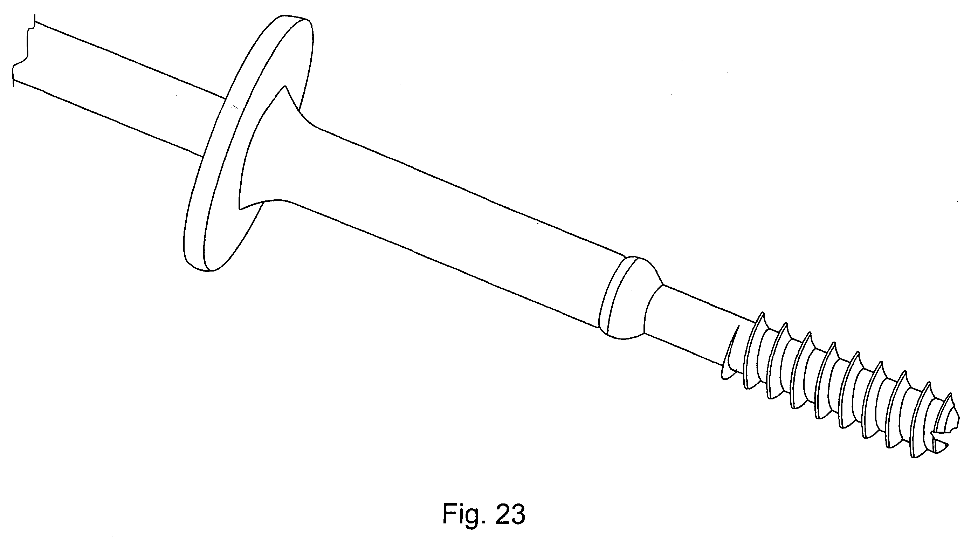

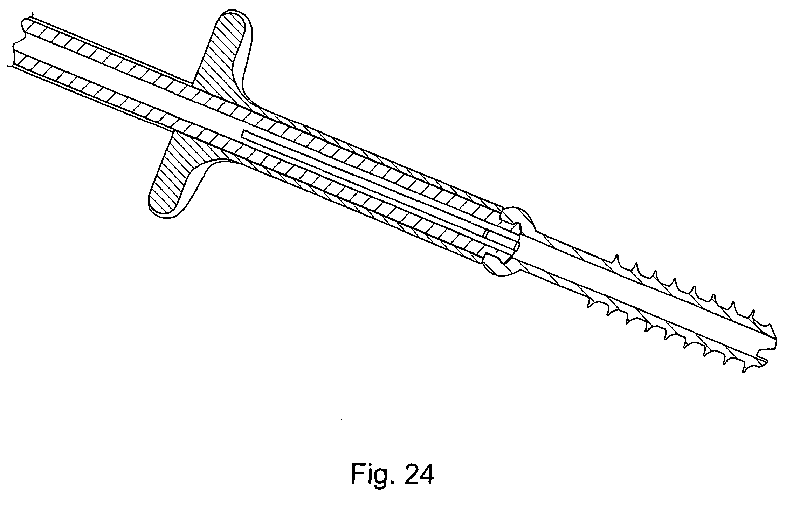

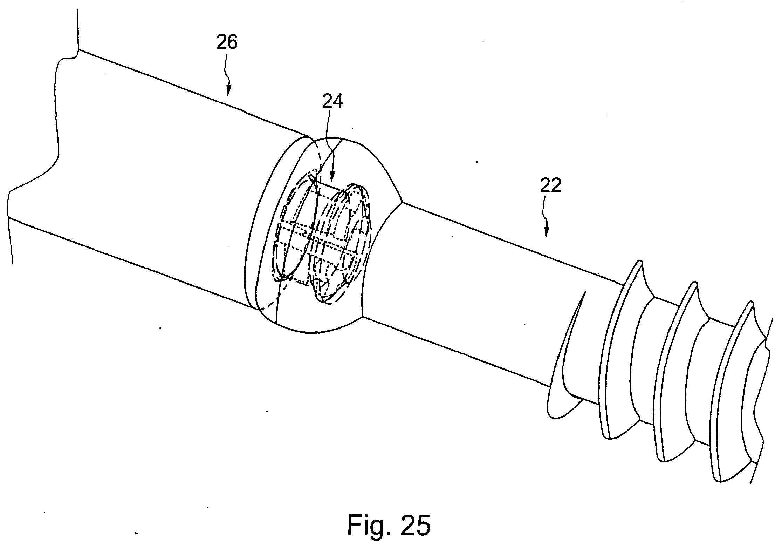

Figures 1 and 11 show a screw holding device 20 engaged with a screw 22. figures 18 to 25 contain eight solid model drawings showings various components of the screw holding device in various state of assembly and in engagement with the screw. The screw holding device 20 has a screw engaging part 24 and a fixation sleeve 26.

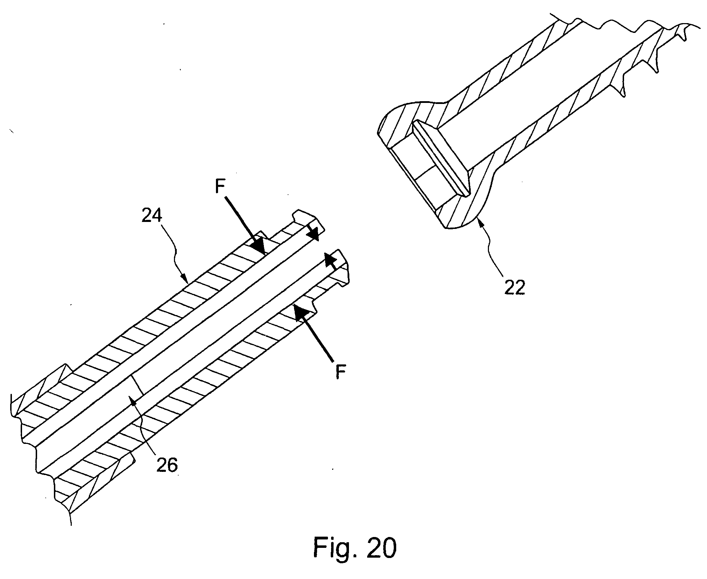

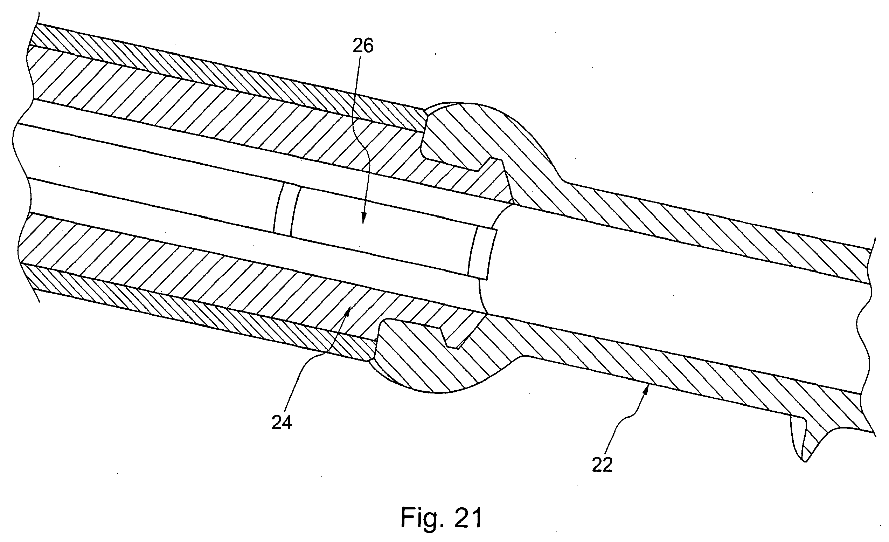

Figure 2 is an enlarged view of the distal portion of the screw engaging part 24 and the screw 22. Figure 2 shows a tip 28 of the screw engaging part 24 inserted into a head 30 of the screw 22. Figure 20 illustrates an open position, in which the sleeve 26 is pulled back with respect to the screw engaging part 24. Thus, it is possible to bend arms 43 radially and to hook into the head of the screw 22. It should be noted that arms or halfs 43 may bend inwardly or may bend outwardly to engage, depending on whether the engaging means of the screw is a recess 36 with a notch 38 like in Figures 1 and 2 where the arms engage from inside, or the engaging means of the screw is an outer recess a direction toward the longitudinal axis (not shown) where the arms 43 engage from outside. Figure 21 illustrates the closed position in which the fixation sleeve or fixation slide 26 is moved forward and blocks the radial movement of the arms 43.

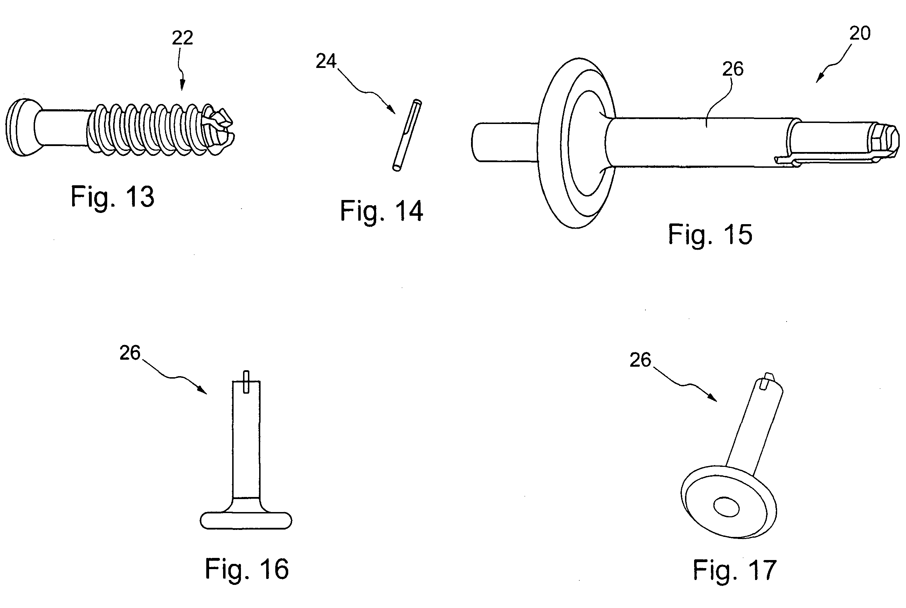

Figure 12 shows a side view of screw 22 and figure 13 shows an isometric view of screw 22. Figure 3 and 4 show cross-sectional views of the screw 22. The screw 22 has the head 30 and shaft 32. The shaft 32 has threads 34. The threads 34 may be selected from the various types of threads known to one skilled in the art. The screw 22 may be used as a bone screw. The head 30 of the bone screw 22 has a recess 36. The recess 36 has six facets; however, the recess 36 may be of any suitable shape known to one skilled in the art. The non-limiting examples of possible shapes are any type of polygon, oval, oblong or star shape. Towards the distal end of the recess 36 and continuous with the six facets is a notch 38. The notch 38 may have a diameter slightly larger then the root diameter of the six facets. The portion of recess 36 distal to notch 38 is tapered to a smaller diameter and connects with a tubular opening 40 that extends along the length of screw 22. It should be noted that the number of facets may also be different from six. The facets may be plane, concave or convex.

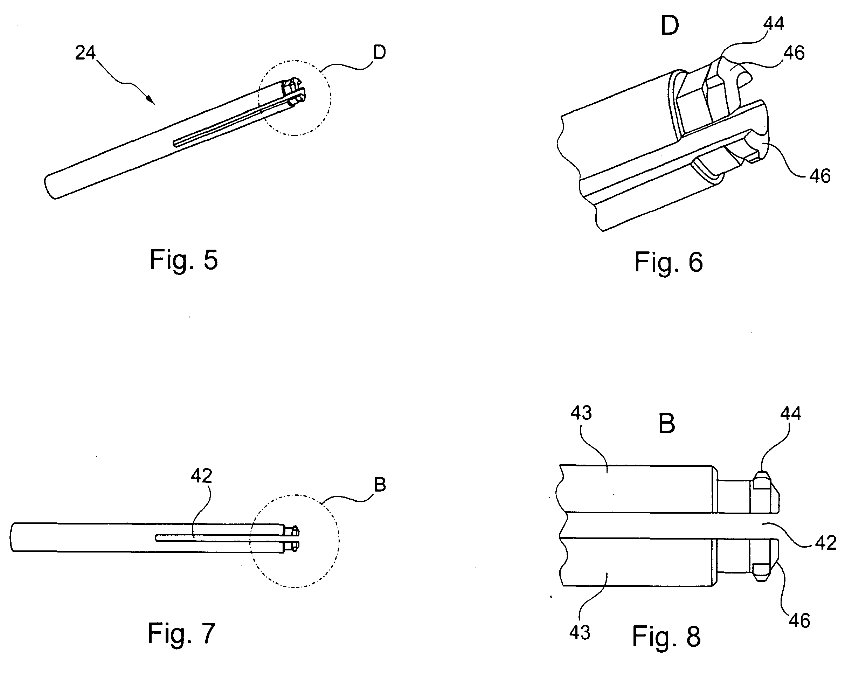

Figures 5, 6, 7, 8 and 14 show screw engaging part 24. The screw engaging part 24 is tubular and has a slot 42 formed towards its distal end. The slot 42 separates the tubular part in two halves 43 that can flex towards and away from each other. The distal ends of the two halves 43 have six facets to match the six facets in the recess 36. The distal ends of the two arms 43 may be of any suitable shape known to one skilled in the art in conjunction with the configuration of recess 36. The mating surfaces of recess 36 and the distal ends of arms 43 are preferably defined by facets, but may be any such configuration that prevents rotational movement between recess 36 and the distal ends of arms 43 when such are connected. A hook 44 is formed on each half 43 of the screw engaging part 24 distal to the six facets. The half 43 may be formed as an axially extending arm like in Figures 5 and 7 or azimutally extending arm (not shown). The hook 44 is shaped to fit in the notch 38 when screw engaging part 24 is inserted in the head 30 of screw 22. The hook 44 and notch 38 can be of any suitable shape. For example, the hook 44 and the notch 38 can have a trapezoidal cross section as seen in figures 4 and 6. Alternatively, hook 44 may be provided on screw 22 and notch 38 may be provided on each arm 43. It should be noted that the hook may also be provided on the screw and the corresponding notch may also be provided on the engaging part. The distal faces 46 of the two halves 43 of the screw engaging part 24 are tapered to form a pointed end. The proximal end of the screw engaging part 24 is suitably formed to attach to drive means, for example, a sonotrode. Alternatively, a handle may be formed on the proximal end of the screw engaging part 24. The screw engaging part 24 may be made from any suitable material. One example of a material suitable for making the screw engaging part 24 is stainless steel, titanium or a titanium alloy.

Figures 9, 10, 16 and 17 show the fixation sleeve 26. The fixation sleeve 26 has a distal end 48, a proximal end 50 and a tubular body 52 connecting the distal end 48 and the proximal end 50. The fixation sleeve 26 is hollow, the hollow portion having a diameter sufficiently large to allow the screw engaging part 24 to smoothly slide in the fixation sleeve 26. The fixation sleeve may be made from any suitable material. One example of a suitable material for the sleeve is stainless steel, titanium or a titanium alloy. Two ribs 54 are formed on the inside surface of the fixation sleeve 26. The ribs 54 are located at the distal end 48 of the fixation sleeve 26. The ribs 54 may be diametrically opposed to each other and may extend from the distal end 48. A disk 56 is formed at the proximal end 50 of the fixation sleeve 26. The disk 56 may be used to push down or pull up the fixation sleeve 26 with respect to the screw engaging part 24. The ribs 56 have a rectangular cross section. Although it is not necessary to have a rectangular cross section, and the ribs may have any suitable shape. The ribs 54 may extend from the distal end 48 of the fixation sleeve. The ribs 56 match with the slot 42 in the screw engaging part 24. When fixation sleeve 26 is connected to screw engaging part 24, ribs 56 are disposed within slots 42 so as to effectively guide the translational movement of fixation sleeve 26 relative to screw engaging part 24. Moreover, the width of ribs 56 is substantially the same as the circumferential width of slots 42, such that when ribs 56 are disposed within slots 42, the circumference of that portion of screw engaging part is substantially complete and rigid.

The screw engaging part 24 and the fixation sleeve 26 are assembled together to form the screw holding device 20 (figure 15). In the assembled state, the Screw engaging part 24 and the fixation sleeve 26 slide relative to each other. The ribs 56 are positioned in the slot 42. The travel of the Screw engaging part 24 relative to the fixation sleeve 26 is limited by the ribs 56 reaching the proximal end of the slot 42. When the fixation sleeve 26 is pulled to the limit in proximal direction, the ribs 56 are at the proximal end of the slot 42 and the two halves 43 of the screw engaging part 24 are able to flex easily towards each other. When the fixation sleeve 26 is moved towards the distal end of the screw engaging part 24, the ribs 56 are in the distal area of the slot 42 and prevent the two halves 43 of the screw engaging part 24 form flexing towards each other. Figure 20 illustrates a position in which fixation sleeve 26 is not connected with screw engaging part 24. Thus, it is possible to flex arms 43 radially and to hook into head 30 of screw 22. It should be noted that arms or halves 43 may bend inwardly or may bend outwardly to engage, depending on whether screw 22 includes recess 36 with notch 38 (as depicted in Figures 1 and 2) or whether screw 22 includes an outer recess in a direction toward the longitudinal axis (not shown) where arms 43 engage from outside. Figure 21 illustrates the closed position in which fixation sleeve 26 is moved forward and prevents radial movement of arms 43.

In use, the fixation sleeve 26 is slid in the proximal direction and the distal tip of the screw engaging part 24 is pressed against the edges of the recess 36 to insert the distal tip of the screw engaging part 24 in the recess 36. When the distal tip of the screw engaging part 24 is pressed against the edges of the recess 36 the two halves 43 of the screw engaging part are pushed towards each other due to the taper formed on the faces 46. With the two halves 43 pushed towards each other, the distal tip of the screw engaging part 24 is able to enter the recess 36. As the tip of the distal part of the screw engaging part 24 travels further in the distal direction in the recess 36, it reaches the notch 38 and the hooks 44 engage in the notch 38 allowing the two halves 43 of the screw engaging part 24 to flex away from each other. The seating of the hooks 44 in the notch 38 prevents the screw holding device 20 from disengaging from the screw 22 accidentally. At this time the fixation sleeve 26 is moved distally as far as possible to prevent the two halves 43 of the screw engaging part 24 from flexing towards each other and thereby making sure that the screw 22 is securely attached to the screw holding device 20. Screw holding device 20 may be detached from screw 22 by moving fixation sleeve 26 proximally along screw engaging part 24. This movement allows arms 43 the ability to flex towards each other. When a sufficient proximal force is then applied to screw engaging part 24, hooks 44 may disengage from notch 38 thereby separating screw holding device 20 from screw 22.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

It should be noted that the term "comprising" does not exclude other elements or steps and the "a" or "an" does not exclude a plurality. Also elements described in association with different embodiments may be combined.

It should also be noted that reference signs in the claims shall not be construed as limiting the scope of the claims.

## Claims

1. An orthopaedic screw holding device for holding a bone screw comprising:
a screw engaging part (24) including a distal end and a proximal end, the screw engaging part having a first portion (43), a second portion (43) and a slot (42) formed at the distal end, the slot (42) extending towards the proximal end thereby forming the first portion (43) in form of a first arm and the second portion (43) in form of a second arm,
an engaging means (28) formed at the distal end, the engaging means being configured to engage a corresponding engaging means (36) of the head of the bone screw, and
a fixation slide (26) slidably engaged with the screw engaging part (24) and movable between a first position and a second position, wherein the fixation slide (26) comprises a sleeve and two ribs (54), wherein the two ribs are formed on the inside surface of the sleeve and are disposed within the slot (42),
wherein the first portion (43) and the second portion (43) of the screw engaging part (24) are movable towards and away from each other when the fixation slide (26) is located in its first position, and
wherein the first portion (43) and the second portion (43) are blocked, so that the orthopaedic screw holding device (20) is lockable with the head (30) of the bone screw (22) when the engaging means (28) engage in the corresponding engaging means (36) of the head (30) of the bone screw (22), and when the fixation slide (26) is located in its second position.

2. Orthopaedic screw holding device of claim 1, further comprising a first facet formed at the distal end, wherein the first facet is configured to engage with a head (30) of the bone screw (22).

3. Orthopaedic screw holding device of any one of claim 1 and 2, wherein the fixation slide (26) is located in its first position towards the proximal end of the screw engaging part (24) and is located in its second position close to the distal end of the screw engaging part (24).

4. Orthopaedic screw holding device of any one of claims 2 to 3, wherein the first facet is configured to engage in the head (30) of the bone screw (22).

5. Orthopaedic screw holding device of any one of claims 1 to 4, wherein the engaging means (24) comprises a protrusion (44) or a hook.

6. Orthopaedic screw holding device of any one of claims 1 to 5, wherein the first arm and the second arm in the first position can be moved towards each other.

7. An orthopaedic screw holding and insertion system comprising:
a bone screw (22) having a head (30), the head having an engaging means (36) a bone screw holding device (20) of any one of claims 1 to 6.

8. Orthopaedic screw holding and insertion system of claim 7, wherein the bone screw further comprises a shank (32) connected to the head (30), and threads (34) formed on at least a portion of the shank (32).

9. Orthopaedic screw holding and insertion system of any one of claims 7 and 8, wherein the engaging means (36) comprises a recess formed in the head, a portion of the recess having a facet formed therein.

10. Orthopaedic screw holding and insertion system of claim 9, wherein the recess having a notch (38) formed in the recess.

## Patentansprüche

1. Orthopädische Schraubenhaltevorrichtung zum Halten einer Knochenschraube, Folgendes umfassend:
einen Schraubeneingriffsteil (24), der ein distales Ende und ein proximales Ende beinhaltet, wobei der Schraubeneingriffsteil einen ersten Abschnitt (43), einen zweiten Abschnitt (43) und einen am distalen Ende gebildeten Schlitz (42) aufweist, wobei sich der Schlitz (42) zum proximalen Ende erstreckt hin, wodurch der erste Abschnitt (43) in Form eines ersten Armes und der zweite Abschnitt (43) in Form eines zweiten Armes gebildet ist,
ein Eingriffsmittel (28), das am distalen Ende gebildet ist, wobei das Eingriffsmittel dafür gestaltet ist, mit einem entsprechenden Eingriffsmittel (36) des Kopfes der Knochenschraube in Eingriff zu stehen, und
ein Befestigungsgleitstück (26), das gleitfähig mit dem Schraubeneingriffsteil (24) in Eingriff steht und zwischen einer ersten Position und einer zweiten Position bewegbar ist, wobei das Befestigungsgleitstück (26) eine Hülse und zwei Rippen (54) umfasst, wobei die zwei Rippen an der Innenoberfläche der Hülse gebildet und in dem Schlitz (42) angeordnet sind,
wobei der erste Abschnitt (43) und der zweite Abschnitt (43) des Schraubeneingriffsteils (24) zueinander hin und voneinander weg bewegbar sind, wenn sich das Befestigungsgleitstück (26) in seiner ersten Position befindet, und
wobei der erste Abschnitt (43) und der zweite Abschnitt (43) derart blockiert sind, dass die orthopädische Schraubenhaltevorrichtung (20) am Kopf (30) der Knochenschraube (22) arretierbar ist, wenn das Eingriffsmittel (28) mit dem entsprechenden Eingriffsmittel (36) des Kopfes (30) der Knochenschraube (22) in Eingriff steht und wenn sich das Befestigungsgleitstück (26) in seiner zweiten Position befindet.

2. Orthopädische Schraubenhaltevorrichtung nach Anspruch 1, ferner eine erste Facette umfassend, die am distalen Ende gebildet ist, wobei die erste Facette dafür gestaltet ist, einen Kopf (30) der Knochenschraube (22) in Eingriff zu nehmen.

3. Orthopädische Schraubenhaltevorrichtung nach einem der Ansprüche 1 und 2, wobei sich das Befestigungsgleitstück (26) in seiner ersten Position zum proximalen Ende des Schraubeneingriffsteils (24) hin befindet und sich in seiner zweiten Position nahe dem distalen Ende des Schraubeneingriffsteils (24) befindet.

4. Orthopädische Schraubenhaltevorrichtung nach einem der Ansprüche 2 bis 3, wobei die erste Facette dafür gestaltet ist, den Kopf (30) der Knochenschraube (22) in Eingriff zu nehmen.

5. Orthopädische Schraubenhaltevorrichtung nach einem der Ansprüche 1 bis 4, wobei das Eingriffsmittel (24) einen Vorsprung (44) oder Haken umfasst.

6. Orthopädische Schraubenhaltevorrichtung nach einem der Ansprüche 1 bis 5, wobei der erste Arm und der zweite Arm in der ersten Position aufeinander zu bewegt werden können.

7. Orthopädisches Schraubenhalte- und -einführsystem, Folgendes umfassend:
eine Knochenschraube (22) mit einem Kopf (30), wobei der Kopf ein Eingriffsmittel (36) aufweist,
eine Knochenschrauben-Haltevorrichtung (20) nach einem der Ansprüche 1 bis 6.

8. Orthopädisches Schraubenhalte- und -einführsystem nach Anspruch 7, wobei die Knochenschraube ferner einen Schaft (32), der mit dem Kopf (30) verbunden ist, und ein Gewinde (34), das an mindestens einem Abschnitt des Schaftes (32) gebildet ist, umfasst.

9. Orthopädisches Schraubenhalte- und -einführsystem nach einem der Ansprüche 7 und 8, wobei das Eingriffsmittel (36) eine Vertiefung umfasst, die im Kopf gebildet ist, wobei ein Abschnitt der Vertiefung eine darin gebildete Facette aufweist.

10. Orthopädisches Schraubenhalte- und -einführsystem nach Anspruch 9, wobei die Vertiefung eine Einkerbung (38) aufweist, die in der Vertiefung gebildet ist.

## Revendications

1. Dispositif de retenue de vis orthopédique destiné à retenir une vis à os, comportant :
une partie de prise de vis (24) comprenant une extrémité distale et une extrémité proximale, la partie de prise de vis ayant une première portion (43), une seconde portion (43) et une fente (42) formée à l'extrémité distale, la fente (42) s'étendant vers l'extrémité proximale en formant ainsi la première portion (43) sous la forme d'un premier bras et la seconde portion (43) sous la forme d'un second bras,
des moyens de prise (28) formés à l'extrémité distale, les moyens de prise étant configurés pour venir en prise avec des moyens de prise (36) correspondants de la tête de la vis à os, et
un coulisseau de fixation (26) en contact de manière coulissante avec la partie de prise de vis (24) et mobile entre une première position et une seconde position, dans lequel le coulisseau de fixation (26) comporte un manchon et deux tétons (54), dans lequel les deux tétons sont formés sur la surface intérieure du manchon et sont disposés à l'intérieur de la fente (42),
dans lequel la première portion (43) et la seconde portion (43) de la partie de prise de vis (24) peuvent se rapprocher et s'écarter l'une de l'autre lorsque le coulisseau de fixation (26) est situé dans sa première position, et
dans lequel la première portion (43) et la seconde portion (43) sont bloquées, de sorte que le dispositif de retenue de vis orthopédique (20) peut être bloqué avec la tête (30) de la vis à os (22) lorsque les moyens de prise (28) viennent en prise dans les moyens de prise (36) correspondants de la tête (30) de la vis à os (22), et lorsque le coulisseau de fixation (26) est situé dans sa seconde position.

2. Dispositif de retenue de vis orthopédique selon la revendication 1, comportant en outre une première facette formée à l'extrémité distale, dans lequel la première facette est configurée pour venir en prise avec une tête (30) de la vis à os (22).

3. Dispositif de retenue de vis orthopédique selon l'une quelconque des revendications 1 et 2, dans lequel le coulisseau de fixation (26) est situé dans sa première position vers l'extrémité proximale de la partie de prise de vis (24) et est situé dans sa seconde position près de l'extrémité distale de la partie de prise de vis (24).

4. Dispositif de retenue de vis orthopédique selon l'une quelconque des revendications 2 à 3, dans lequel la première facette est configurée pour s'engager dans la tête (30) de la vis à os (22).

5. Dispositif de retenue de vis orthopédique selon l'une quelconque des revendications 1 à 4, dans lequel les moyens de prise (24) comportent une saillie (44) ou un crochet.

6. Dispositif de retenue de vis orthopédique selon l'une quelconque des revendications 1 à 5, dans lequel le premier bras et le second bras dans la première position peuvent être rapprochés l'un de l'autre.

7. Système d'insertion et de retenue de vis orthopédique comportant :
une vis à os (22) ayant une tête (30), la tête ayant des moyens de prise (36) et un dispositif de retenue de vis à os (22) selon l'une quelconque des revendications 1 à 6.

8. Système d'insertion et de retenue de vis orthopédique selon la revendication 7, dans lequel la vis à os comporte en outre une tige (32) reliée à la tête (30), et des filets (34) formés sur au moins une portion de la tige (32).

9. Système d'insertion et de retenue de vis orthopédique selon l'une quelconque des revendications 7 et 8, dans lequel les moyens de prise (36) comportent un évidement formé dans la tête, une portion de l'évidement ayant une facette formée dans celle-ci.

10. Système d'insertion et de retenue de vis orthopédique selon la revendication 9, dans lequel l'évidement a une entaille (38) formée dans l'évidement.
